# EUROPEAN PATENT APPLICATION

(11) **EP 2 156 857 A2**
(43) Date of publication of application: **24.02.2010**
(21) Application number: 09014271.2
(22) Date of filing: 08.04.2008
(51) Int. Cl.: A61M 5/145, A61M 5/31

(54) **Universal syringe**

(30) Priority: 11.04.2007 US 921474 P
(62) Divisional of application: 08742651.6
(71) Applicant: Mallinckrodt Inc., Hazelwood, MO 63042 (US)
(72) Inventor: Fago, Frank M., Ohio 45040 (US); Neer, Charles S., Cincinnati Ohio 45202 (US)
(74) Representative: Chettle, Adrian John

(57) **Abstract**

Syringes for use with medical fluid injectors. In certain aspects, the syringe includes a body having a longitudinal axis, a forward end, and a rearward end. At least one syringe mating section is coupled to the body of the syringe and is adapted to operatively engage an injector. In some embodiments, the syringe mating section includes first, second, and third flanges extending outwardly from the body along a plane substantially perpendicular to the longitudinal axis. In some embodiments, the syringe mating section includes a first flange having a cross-section of a parallelogram and a second flange having a tapered surface. In some embodiments, the syringe mating section includes a flange having an L-shaped cross-section. Other embodiments of the syringe mating section may include yet other appropriate designs.

## Description

### FIELD OF THE INVENTION

The present invention relates generally to syringes and injectors for injecting medical fluids, and more particularly, to the mating sections of such syringes that are adapted to be engaged with complementary mating sections of such injectors.

### BACKGROUND

This section is intended to introduce the reader to various aspects of art that may be related to various aspects of the present invention, which are described and/or claimed below. This discussion is believed to be helpful in providing the reader with background information to facilitate a better understanding of the various aspects of the present invention.
Accordingly, it should be understood that these statements are to be read in this light, and not as admissions of prior art.

During many medical procedures, various fluids are injected into patients for purposes of diagnosis or treatment. An example of one such fluid is contrast media used to enhance angiography, MRI, or CT imaging. Injectors used in these procedures tend to be automated devices that expel fluid from a syringe, through a tube, and into the patient. Often, the syringes will already be prefilled with fluid (e.g., with contrast media, in any of a number of appropriate volumes) when purchased by the user.

The syringes used in above-described imaging procedures generally include a barrel having a hollow interior and a discharge tip, and a syringe plunger disposed within the barrel. In many such injectors, the syringes are replaceable. For instance, to replace a syringe, a portion of the injector that facilitates coupling between the syringe plunger and a plunger drive ram of the injector may be moved, the used syringe may then be removed, and a fresh syringe may subsequently be mounted to the injector.

To facilitate installation on an injector, each syringe includes syringe mating sections that are adapted to mate with injector mating sections located on the injector. The syringe mating sections tend to be disposed at least partially circumferentially around the rearward end of the syringe. These syringe mating sections generally have a shape or contour that can engage and/or be engaged by the injector mating sections in a locking engagement therewith. In some injectors, the injector mating sections are located in a syringe-receiving portion of the injector and tend to be disposed partially circumferentially around the syringe-receiving portion of the injector. In many instances, the syringe may be inserted into the syringe-receiving portion of the injector in a manner such that the syringe mating sections and injector mating sections have little or no contact with one another. Once the syringe has been inserted such that the syringe mating sections are positioned in desired locations relative to the injector mating sections, the syringe may be rotated relative to the injector to bring the syringe mating sections and injector mating sections into locking engagement with one another.

A drawback of conventional injectors is that different injectors (e.g., those made by different companies) are equipped with differently-shaped injector mating sections, such that particular syringe mating sections of a given syringe may not be compatible with a number of injectors. Thus, the users of those injectors may be relegated to using only syringes that are compatible with the particular injector(s) they use. This is undesirable because different companies produce different syringes (prefilled versus unfilled, contrast media versus other fluids, different fill volumes, etc.), and thus, a consumer may be barred from using the particular syringe and/or medical fluid he/she desires simply because a given syringe many not be compatible with the injector.

A drawback to current syringes used with medical fluid injectors is a function of forces generated during injection procedures. For example, the force exerted by pressures on a 200 ml syringe during a CT injection can exceed 800 lbs on certain injector mating sections. This force traditionally has been resolved primarily by shear resistance of the injector mating sections on the injector. One manner of increasing the shear strength is to form the syringe from a material that has a high shear resistance. Unfortunately, many materials that are better suited for prefilled syringe fluid contact requirements may not be optimal for shear resistance.

### SUMMARY

Certain aspects of the invention are set forth below to provide the reader with a brief summary of certain forms the invention might take. These aspects are not intended to limit the scope of the invention. Indeed, the invention may encompass a variety of features and aspects that may not be set forth below.

In one aspect, a mechanism is provided for supporting a syringe on an injector (e.g., on a face plate thereof or on a front wall of the injector housing). The mechanism includes an injector mating section of the injector that is releasably engageable with a syringe mating section of the syringe. In some embodiments, the mechanism may be characterized as an interlocking mechanism that may be activated and/or released upon rotation of a rearward portion of the syringe relative to the injector. For instance, the injector mating section may include a plurality of slots for receiving a plurality of syringe flanges (e.g., the syringe mating section) located toward the rearward end of the syringe. As such, the syringe flanges can be inserted into the slots, and the syringe can be subsequently rotated to engage the syringe flanges behind injector flanges of the injector mating section.

In some embodiments, the syringe mating section includes first and second flanges extending outwardly from the body of the syringe, away from a longitudinal axis of the body, and along a plane substantially perpendicular to the longitudinal axis. The syringe mating section of some embodiments may include yet a third flange that extends outwardly from the body, away from the longitudinal axis, and along a plane substantially perpendicular to the longitudinal axis.

Any of a number of designs may be appropriate for the syringe mating section of the syringe. For instance, in some embodiments, the syringe mating section may include a flange having a cross-section in the form of a parallelogram. As another example, the syringe mating section of some embodiments may additionally or alternatively include a flange that has a tapered surface relative to the longitudinal axis of the syringe body. In some embodiments, the syringe mating section may include a flange that exhibits an L-shaped cross-section that may or may not exhibit any discontinuities along its length.

Syringes incorporating syringe mating sections of the invention may be found compatible with multiple injectors, thus increasing options for consumers of such syringes. While at least some of the syringe mating sections of the invention are designed to be compatible with more than one injector, their shapes may be found to increase an overall area of such syringe mating sections relative to conventional syringe mating sections. Thus, syringes of the present invention may be able to withstand greater shear forces during an injection procedure than previous syringes. As such, syringes of the present invention may reduce syringe failure rates.

Various refinements exist of the features noted above in relation to the various aspects of the present invention. Further features may also be incorporated in these various aspects as well. These refinements and additional features may exist individually or in any combination. For instance, various features discussed below in relation to one or more of the illustrated embodiments may be incorporated into any of the above-described aspects of the present invention alone or in any combination. Again, the brief summary presented above is intended only to familiarize the reader with certain aspects and contexts of the present invention without limitation to the claimed subject matter.

### BRIEF DESCRIPTION OF THE FIGURES

Fig. 1 is a perspective view of an injector head of an injector, including a syringe attached thereto and a face plate;

Fig. 2 is a cross-sectional view of the injector head, face plate, and syringe of Fig. 1 taken along lines 2-2;

Fig. 3A is a perspective view of a syringe, depicting one embodiment of a syringe mating section;

Figs. 3B and 3C are cross-sectional views of the syringe of Fig. 3A in engagement with a face plate of a first injector;

Figs. 3D and 3E are cross-sectional views of the syringe of Fig. 3A in engagement with a face plate of a second injector;

Fig. 4A is a perspective view of a syringe, depicting another embodiment of a syringe mating section;

Figs. 4B and 4C are cross-sectional views of the syringe of Fig. 4A in engagement with a face plate of a first injector;

Figs. 4D and 4E are cross-sectional views of the syringe of Fig. 4A in engagement with a face plate of a second injector;

Fig. 5A is a perspective view of a syringe, depicting yet another embodiment of a syringe mating section;

Figs. 5B and 5C are cross-sectional views of the syringe of Fig. 5A in engagement with a face plate of a first injector;

Figs. 5D and 5E are cross-sectional views of the syringe of Fig. 5A in engagement with a face plate of a second injector;

Fig. 6A is a perspective view of a syringe, depicting still another embodiment of a syringe mating section;

Figs. 6B and 6C are cross-sectional views of the syringe of Fig. 6A in engagement with a face plate of a first injector;

Figs. 6D and 6E are cross-sectional views of the syringe of Fig. 6A in engagement with a face plate of a second injector;

Fig. 7A is a perspective view of a syringe, depicting still yet another embodiment of a syringe mating section;

Figs. 7B and 7C are cross-sectional views of the syringe of Fig. 7A in engagement with a face plate of a first injector; and

Figs. 7D and 7E are cross-sectional views of the syringe of Fig. 7A in engagement with a face plate of a second injector.

### DETAILED DESCRIPTION OF EXEMPLARY EMBODIMENTS

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

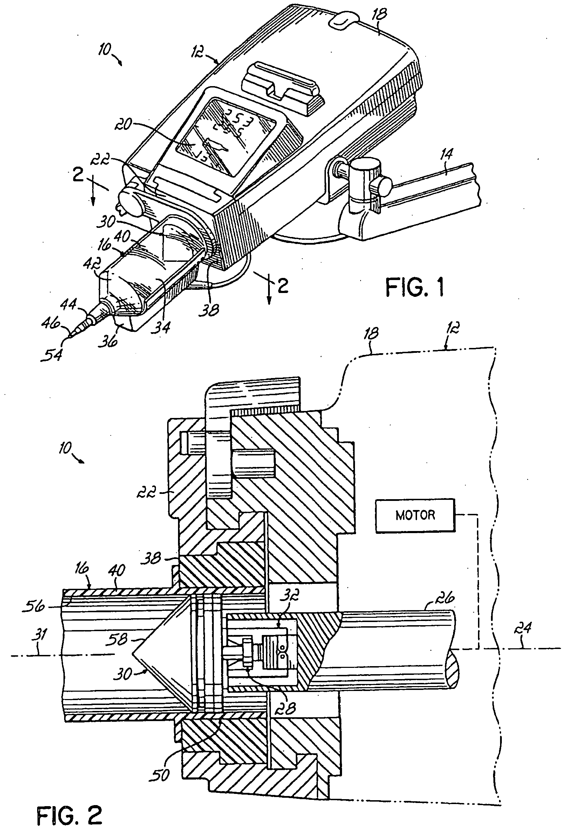

Referring to Figs. 1-2, an injector 10 is depicted, having an injector head 12 attached to an arm 14, which in turn may be mounted to a ceiling, wall, or floor joint (not shown). This allows for motion of the injector head 12, for example, so that it may be positioned to receive and load a syringe 16 and to inject fluids into a subject (not shown). Surrounding the inner mechanism of the injector 10 is an injector housing 18. This housing 18 includes a display panel 20. The display panel 20 aids the operator of the injector 10 in monitoring amounts of fluid injected into a subject.

Toward the forward end of the injector housing 18, positioned at least generally between the injector 10 and the syringe 16, is a face plate 22. The face plate 22 may be slidable along a plane perpendicular to a longitudinal axis 24 of the motion of a plunger drive ram 26 of the injector 10. The purpose of this face plate 22 is to facilitate connection between the injector housing 18 and the syringe 16, and in certain embodiments, to facilitate disengagement of a coupling element 28 of a syringe plunger 30 from a coupling mechanism 32 of the plunger drive ram 26. For example, a syringe 16 may be loaded into and coupled to the injector 10, such as at the face plate 22, by engaging respective mating sections 48 on the outer surface of the syringe 16 and on the face plate 22. In certain embodiments, the disengagement may be affected by moving the face plate 22 transverse to the longitudinal axis 24 of the plunger drive ram 26. In addition or as an alternative to transverse motion, face plate 22 may be capable of pivotal motion to enable rear loading a new syringe into a pressure jacket 34 of the injector and/or to enable unloading and removal of a syringe from the pressure jacket 34 after the face plate 22 has been moved to disengage the rearwardly extending coupling element 28 of the syringe plunger 30 and the coupling mechanism 32 of the plunger drive ram 26.

A pressure jacket 34, which may be transparent, may extend outwardly from the face plate 22, and houses the syringe 16. The syringe 16 and pressure jacket 34 are constructed such that they collectively withstand injection pressures created by the injector 10 during an injection operation. It should be noted that some injectors of the invention may not include a face plate (e.g., 22) and/or a pressure jacket (e.g., 34). For instance, an injector equipped with a pressure jacket may not be desired by some users in low pressure injection applications.

A cradle 36 is operatively connected to the injector 10. In the illustrated embodiment, the cradle 36 extends from the front surface 38 of the face plate 22, and supports the syringe 16 and pressure jacket 34. The cradle 36 may include a mechanism to warm the contents of the syringe 16, thus allowing the contents of a syringe 16 to be maintained at a particular desired temperature while the syringe 16 is attached to the injector 10. The syringe 16 is held in proximity with the cradle 36 such that the medical fluid (e.g., contrast media, saline, etc.) within the syringe 16 is warmed.

The syringe 16 includes an exterior cylindrical body 40, which, at its forward end, is integral with a conical front wall section 42. A neck 44, terminating in discharge tip 46, extends forwardly from and is integral with the front wall 42. The body 40 of the syringe 16 snugly interfaces with the interior walls of the pressure jacket 34. This syringe 16 includes a syringe mating section 48, which may be in the form of a radially outwardly extending annular flange, and which is positioned in a plane perpendicular to the axis of, and integral with, the rear end 50 of the cylindrical body 40 of the syringe 16. The syringe mating section 48 is arranged, when the syringe 16 is located within the pressure jacket 34, to align with cooperating mating sections located on the rear end 50 of the pressure jacket 34, and/or to interface with mating sections 52 located in the face plate 22. In this manner, the syringe 16 and pressure jacket mating sections or injector mating sections 52 facilitate the connection of the syringe 16 to the injector 10.

The neck 44 of the discharge tip 46 contains an orifice 54 in its remote end, which communicates with an internal syringe cavity 56 formed within the neck 44, the conical front wall 42, and the body 40 of the syringe 16. The rear end of the cavity 56 is further defined by a forward facing conical surface 58 of the syringe plunger 30. The conical surface 58 is preferably of a slope, which substantially conforms to the slope of the interior of the conical front wall 42. The syringe plunger 30 is snugly slidable within the body 40 of the syringe 16 such that the cavity 56 is of variable volume.

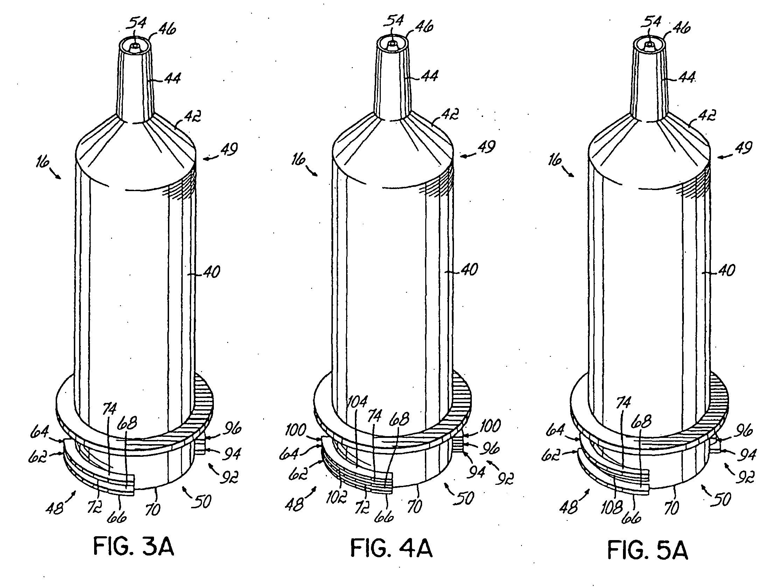

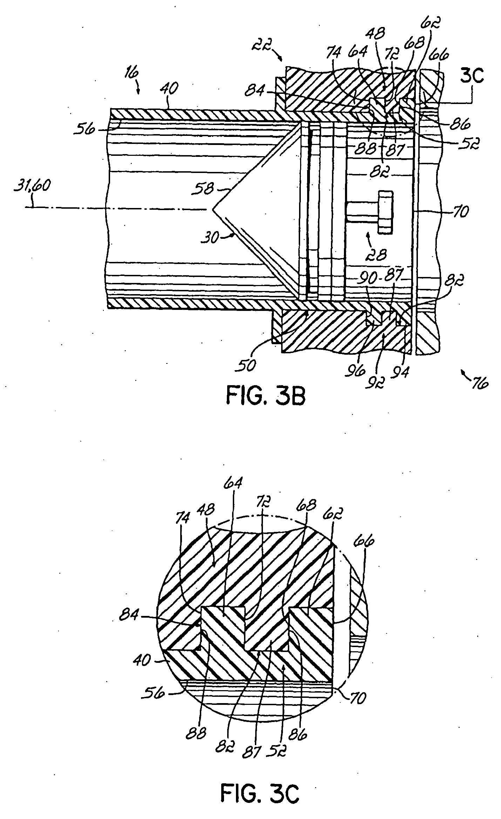

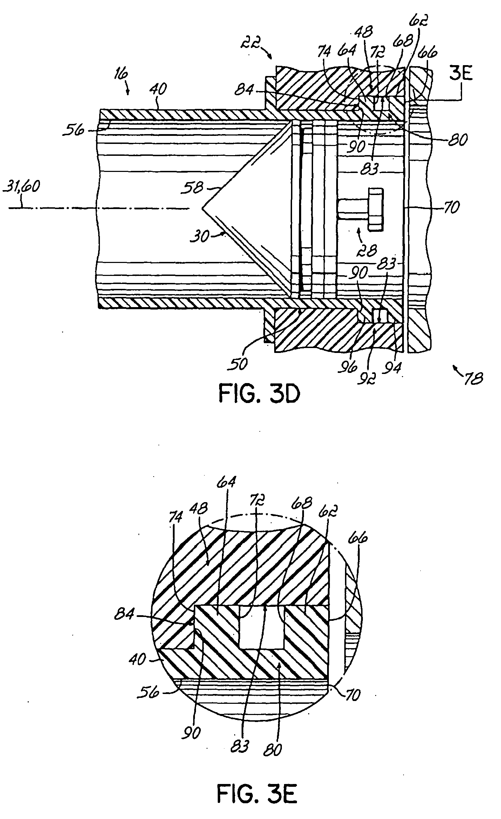

Referring now to Figs. 3A-3E, a syringe 16 in accordance with principles of the present invention is shown. The body 40 of the syringe 16 has a longitudinal axis 60 and includes a forward end 49 and a rearward end 50. At least one syringe mating section (a first syringe mating section 48) is coupled to the body 40. The syringe mating section 48 (and those of other embodiments described herein) "coupled" to the body 40 may refer to a separate syringe mating section that is physically attached to the body, such as by bonding, adhesives, or any other suitable method, or may refer to a syringe mating section that is integrally formed with the body. The syringe mating section 48 is adapted to engage an injector 10 and includes a first flange 62 and a second flange 64, each extending outwardly from the body 40 along a plane substantially perpendicular to the longitudinal axis 60 of the body 40. In particular, the first flange 62 includes a rearward surface 66 and a forward surface 68. The rearward surface 66 of the first flange 62 may be flush or substantially flush with a proximal end 70 of the syringe 16. The second flange 64 is located distally of the first flange 62 and includes a rearward surface 72 and a forward surface 74. The rearward surface 72 of the second flange 64 is spaced from the forward surface 68 of the first flange 62. It should be noted that the first and second flanges 62, 64 may be positioned at other locations (e.g., remote from the proximal end) along the body 40 in other embodiments of the syringe 16. Further, each of the first and second flanges 62, 64 has a generally square or rectangular cross-section in the illustrated embodiment. However, this is merely illustrative, and flanges having cross-sections of other shapes may be used.

The first syringe mating section 48 illustrated in Figs. 3A-3E exhibits an arcuate length generally equal to approximately one quarter of the circumference (i.e., about 90°) of the body 40. However, other embodiments of the syringe mating section 48 may exhibit other appropriate arcuate lengths that may be greater or less than about 90°.

As can be seen in Figs. 3B through 3E, the syringe 16 including the first syringe mating section 48 can be mounted to injectors of more than one design. A first injector 76, having a face plate 22 with a first injector mating section 52, is depicted in Figs. 3B-3C, and a second injector 78, having a face plate 22 with a second injector mating section 80, is depicted in Figs. 3D-3E. The injector mating section 52 of the first injector 76 (Fig. 3B-3C) is defined by a contour 82 that is complementary to the first syringe mating section 48. In other words, the contour 82 of the injector mating section 52 is closely aligned with and substantially matches a contour 84 defining the first syringe mating section 48. In particular, the injector mating section 52 has a first groove 86 and a second groove 88 defined in a surface 38 of the face plate 22, with a flange 87 located therebetween. When the syringe 16 is operatively connected to the face plate 22, the first flange 62 is received within the first groove 86, and the second flange 64 is received within the second groove 88. And thus, the flange 87 of the injector mating section 52 is positioned between the first flange 62 and second flange 64 when the first syringe mating section 48 and the injector mating section 52 are in an engaged relationship. Again, those skilled in the art will recognize that cross-sectional shapes of the injector mating section grooves 86, 88 and flange 87 may be other than the square or rectangular shapes shown in the illustrated embodiment, and may be dependent on the shape of the flanges 62, 64 of the syringe mating section 48.

The injector mating section 80 of the second injector 78 (Fig. 3D-3E) is defined by a contour 83 that cooperates with the first syringe mating section 48, but is not completely complementary to the first syringe mating section 48. In other words, the contour 83 does not substantially match the contour 84 of the first syringe mating section 48. However, the contour 83 is such that the injector mating section 80 does receive the first and second flanges 62, 64 in such manner that the first syringe mating section 48 and injector mating section 80 engage to operatively connect the syringe 16 to the second injector 78. In particular, the injector mating section 80 includes a single groove 90 having a dimension parallel to the longitudinal axis 60 of the syringe 16 that receives both the first flange 62 and the second flange 64 of the first syringe mating section 48. While the single groove 90 of the illustrated embodiment is depicted as having a rectangular cross-section, those skilled in the art will recognize that a single groove of any alternate shape may suffice, provided it can receive the syringe mating section to form a connection between the syringe 16 and second injector 78. Thus, the syringe 16 of Figs. 3A-3E can be used with different injectors 76, 78 having different injector mating sections 52, 80.

Still referring to Figs. 3A-3E, each of the first and second flanges 62, 64 extends only partially circumferentially around the rearward end 50 of the body 40. This configuration allows the syringe 16 to be loaded into the face plate 22 of the injector 10. The syringe 16 of the illustrated embodiment may further include a second syringe mating section 92 operatively coupled to the body 40 and positioned in an annular location substantially opposite the first syringe mating section 48. Thus, the first and second syringe mating sections 48, 92 combined, encompass an arcuate length generally equal to approximately one half of the circumference (i.e., about 180°) of the body 40 (although it will be recognized by those skilled in the art that the mating sections 48, 92 may encompass an arcuate length other than about one-half of the circumference of the body 40, and that there may be more than first and second mating sections). Like the first syringe mating section 48, the second mating section 92 includes first and second flanges 94, 96 extending outwardly from the body 40 along a plane substantially perpendicular to the longitudinal axis 60 of the body 40.

Still referring to Figs. 3A-3E, to load the syringe 16 into either the first injector 76 or the second injector 78, the first and second flanges 62, 64 of the first syringe mating section 48 and the first and second flanges 94, 96 of the second syringe mating section 92 are aligned with gaps (not shown) between first and second injector mating sections. The syringe 16 is then inserted into an opening in the face plate 22 until the first and second syringe mating sections 48, 92 are aligned with the injector mating sections of the face plate 22. The syringe 16 is then rotated to position the first and second syringe mating sections 48, 92 within, and in confronting relationship with, the injector mating sections 52 of the first injector 76 or the injector mating sections 80 of the second injector 78. In this position, the syringe 16 is connected to the face plate 22.

As can be seen in the embodiment illustrated in Figs. 3A-3E, each of the flanges 62, 64, 94, 96 does not exhibit any discontinuity along its length. However, it will be recognized by those skilled in the art that one or more of the flanges 62, 64, 94, 96 could exhibit at least one discontinuity along its length. Such a discontinuity could be, for example, a gap through the flange(s), a notch cut into the flange(s), or an undulating contour of the flange(s).

Each of the flanges 62, 64, 94, 96 shown in Figs. 3A-3E does not have a cavity defined therein. In other words, as opposed to a discontinuity, which forms a disruption between the forward and rearward ends of a particular flange of the syringe mating section, a cavity does not form any disruption in the forward and rearward ends of a given flange, but does form a hollowed portion of the flange. It should be noted that other embodiments may include one or more flanges having at least one cavity defined therein.

Still referring to Figs. 3A-3E, the syringe mating sections 48, 92 are adapted to operatively engage the face plate 22 of the injector 10. However, those skilled in the art will recognize that engagement with a face plate is not necessary to the present invention and that the syringe mating sections 48, 92 may be utilized to operatively engage another portion an injector (e.g., a housing of an injector).

With further reference to Figs. 3A-3E, the syringe mating sections 48, 92 may allow the syringe 16 to withstand greater shear forces than conventional syringe mating sections. This feature may reduce the failure rate of such syringes as compared to conventional syringes. As described above, a drawback of conventional syringes is that many materials that are used in making prefilled syringes are inadequate to withstand shear forces generated during injection procedures. Thus, the syringe mating sections can be sheared from the body of the syringe during an injection, resulting in the need to replace the syringe and repeat the injection procedure. Previously, to cope with this problem, the sizes of the syringe mating sections have been increased in order to increase the area in shear (i.e., to increase the surface area of contact between the syringe mating section and the injector mating section in the direction of the injection forces, e.g., in a direction along the longitudinal axis of the syringe). However, when forming such syringe mating sections during injection molding, such larger sections are weakened by the cooling process as compared to smaller sections. Thus, one aspect of the present invention results from the recognition that the size of syringe mating sections 48, 92 can be reduced (thereby improving molding of the syringe mating section) while increasing the overall area of the syringe mating sections 48, 92 in shear (thereby reducing the failure rate of syringes). Thus, the contours 84 of the syringe mating sections 48, 92 of the syringe 16 of the illustrated embodiment result in the syringe mating sections 48, 92 having an increased area in shear as compared to the area of syringe mating sections of conventional syringes. For example, syringes produced by Medrad, Inc. (of Pittsburgh, PA) for the Medrad Envision and Medrad Vistron injectors have syringe mating sections having about 0.127 square inch of area for shear resistance. This is due to the fact that only the distal end of a syringe mating section has a surface that is in contact with a surface of an injector mating section. However, the syringe mating section 48 of the embodiment illustrated in Figs. 3A-3E has two such surfaces (i.e., the forward surface 68 of first flange 62 and the forward surface 74 of second flange 64). Thus, it may have about 0.246 square inch of area for shear resistance, for example. Additionally, the syringe mating sections of the conventional syringes are weaker due to the configuration of the flanges of the syringe mating section, which include cavities therein. However, the syringe mating section 48 of the embodiment illustrated in Figs. 3A-3E does not include any such cavities (i.e., are solid first and second flanges 62, 64). Thus, the embodiment of the syringe mating sections of Figs. 3A-3E is easier to mold due to the reduced thickness of each lug, while the shear resistance is actually increased. It will be recognized by those skilled in the art that the particular contours of the syringe mating sections shown in Figs. 3A-3E are merely exemplary, and that any contour or shape may be used which increases the overall area in shear.

The area of a syringe of the invention that is dedicated to shear resistance may be at least 0.13 square inch in some embodiments, at least 0.14 square inch in some embodiments, at least 0.15 square inch in some embodiments, at least 0.16 square inch in some embodiments, at least 0.17 square inch in some embodiments, at least 0.18 square inch in some embodiments, at least 0.19 square inch in some embodiments, at least 0.20 square inch in some embodiments, at least 0.21 square inch in some embodiments, at least 0.22 square inch in some embodiments, at least 0.23 square inch in some embodiments, at least 0.24 square inch in some embodiments, and at least 0.25 square inch in some embodiments. Other embodiments may exhibit yet other appropriate shear resistance areas.

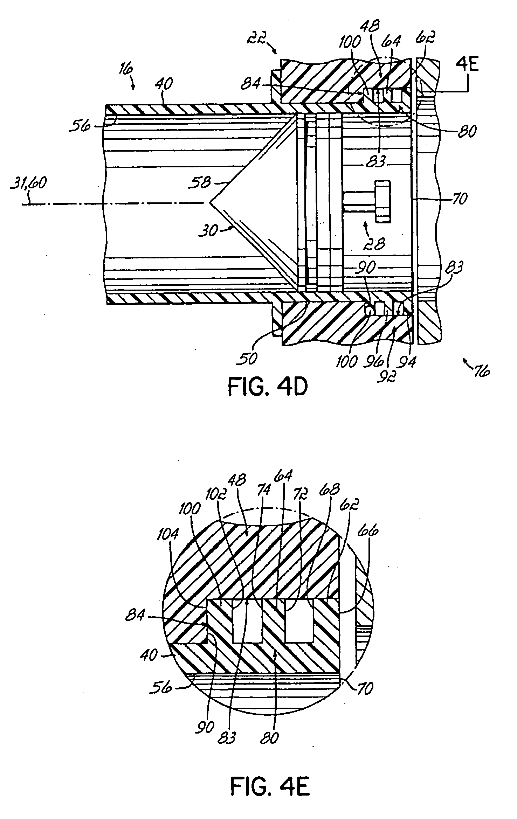

Referring now to Figs. 4A-4E, a second embodiment of a syringe 16 having at least one syringe mating section (a first syringe mating section 48) is shown. This first syringe mating section 48 is adapted to engage an injector 10, and includes a first flange 62, a second flange 64, and a third flange 100, each extending outwardly from a body 40 of the syringe 16 along a plane substantially perpendicular to a longitudinal axis 60 of the body 40. In particular, the first flange 62 includes a rearward surface 66 and a forward surface 68. The rearward surface 66 of the first flange 62 may be flush or substantially flush with a proximal end 70 of the syringe 16. The second flange 64 is located distally of the first flange 62 between the first and third flanges 62, 100 and includes a rearward surface 72 and a forward surface 74, with the rearward surface 72 being spaced from the forward surface 68 of the first flange 62. The third flange 100 is located distally of the second flange 64 and includes a rearward surface 102 and a forward surface 104, with the rearward surface 102 being spaced from the forward surface 74 of the second flange 64. Other embodiments of the syringe 16 may exhibit other appropriate positionings of the first, second, and third flanges 62, 64, 100 along the surface of the body 40. Each of the first, second, and third flanges 62, 64, 100 is shown as having a generally square or rectangular cross-section in the illustrated embodiment. However, this is merely illustrative, and flanges having cross-sections of other shapes may be used.

The first syringe mating section 48 illustrated in Figs. 4A-4E exhibits an arcuate length generally equal to approximately one quarter of the circumference (i.e., about 90°) of the body 40. However, other embodiments of the syringe mating section 48 may exhibit other appropriate arcuate lengths that may be greater or less than about 90°.

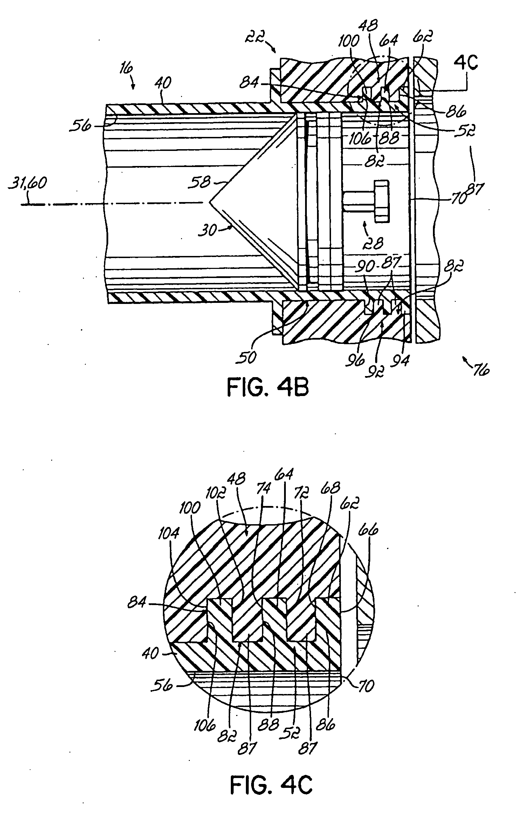

As can be seen in Figs. 4B through 4E, the syringe 16 including the first syringe mating section 48 can be mounted to injectors having different designs. A first injector 76, having a face plate 22 with a first injector mating section 52, is depicted in Figs. 4B-4C, and a second injector 78, having a face plate 22 with a second injector mating section 80, is depicted in Figs. 4D-4E. The injector mating section 52 of the first injector 48 (Figs. 4B-4C) is defined by a contour 82 that is complementary to the first syringe mating section 48. In other words, the contour 82 of the injector mating section 80 is closely aligned with and substantially matches a contour 84 defining the first syringe mating section 48. In particular, the injector mating section 52, as illustrated, includes a first groove 86, a second groove 88, and a third groove 106 disposed in a surface 38 of the face plate 22. When the syringe 16 is operatively connected to the face plate 22, the first flange 62 is received within the first groove 86, the second flange 64 is received within the second groove 88, and the third flange 100 is received within the third groove 106. As with the embodiment described above, those skilled in the art will recognize that cross-sectional shapes of the injector mating section grooves 86, 88, 106 may be other than the square or rectangular shapes shown in the illustrated embodiment, and may be dependent on the shape of the flanges 62, 64, 100 of the syringe mating section 48.

The injector mating section 80 of the second injector 78 (Figs. 4D-4E) is defined by a contour 83 that cooperates with the first syringe mating section 48, but is not completely complementary to the first syringe mating section 48. In other words, the contour 83 does not substantially match the contour 84 of the first syringe mating section 48. However, the contour 83 is such that the injector mating section 80 does receive the first, second, and third flanges 62, 64, 100 in such manner that the first syringe mating section 48 and injector mating section 80 engage to operatively connect the syringe 16 to the second injector 78. In particular, the injector mating section 80 includes a single groove 90 having a dimension parallel to the longitudinal axis 60 of the syringe 16 that receives the first flange 62, the second flange 64, and the third flange 100 of the first syringe mating section 48. Like that described above in the previous embodiment, while the single groove 90 of the illustrated embodiment of Fig. 4C is depicted as having a rectangular cross-section, those skilled in the art will recognize that a single groove of any alternate shape may suffice, provided it can receive the syringe mating section 48 to form a connection between the syringe 16 and second injector 78. Thus, the syringe 16 of the embodiment of Figs. 4A-4E can be used with different injectors 76, 78 having different injector mating sections 52, 80, thereby increasing the versatility of the syringe 16.

Like the embodiment illustrated in Figs. 3A-3E above, in the syringe embodiment of Figs. 4A-4E, each of the flanges 62, 64, 100 may extend only partially circumferentially around the body 40 of the syringe 16. The syringe 16 of Figs. 4A-4E includes a second syringe mating section 92 operatively coupled to the body 40 and positioned substantially opposite the first syringe mating section 48. Thus, as illustrated, the first and second syringe mating sections 48, 92 combined, encompass an arcuate length generally equal to approximately one half of the circumference (i.e., about 180°) of the body 40 (although it will be recognized by those skilled in the art that the mating sections 48, 92 may encompass an arcuate length other than about one-half of the circumference of the body 40, and that there may be more than first and second mating sections). And while the illustrated embodiment does not depict a discontinuity or cavity in the syringe mating sections 48, 92, those skilled in the art will recognize that the syringe mating sections 48, 92 may exhibit such a discontinuity and/or cavity. Further, while the syringe mating section 48 is shown as engaging a face plate 22, it may be adapted to engage other portions of an injector, such as a housing of an injector.

The syringe mating sections 48, 92 of the embodiment of Figs. 4A-4E may allow the syringe 16 to withstand greater shear forces than conventional syringe mating sections. This may reduce the failure rate of the syringe 16 as compared to conventional syringes. For example, a syringe mating section 48 of the embodiment illustrated in Figs. 4A-4E may have about 0.205 square inch of area for shear resistance.

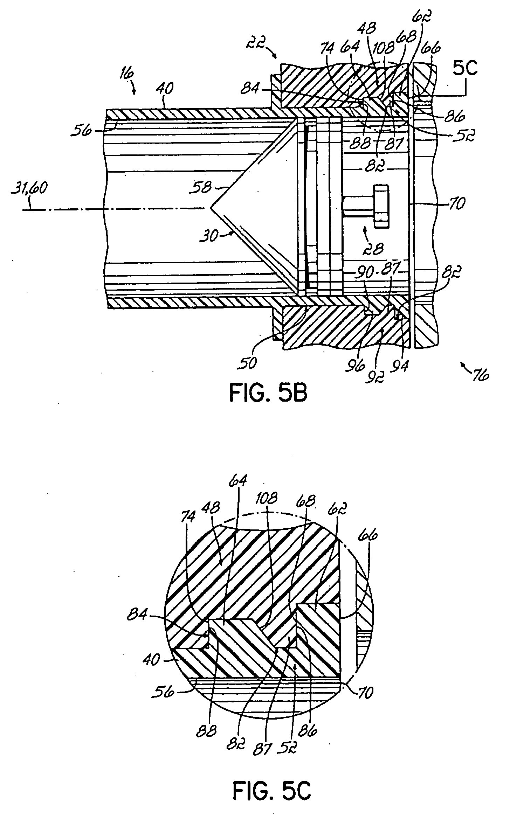

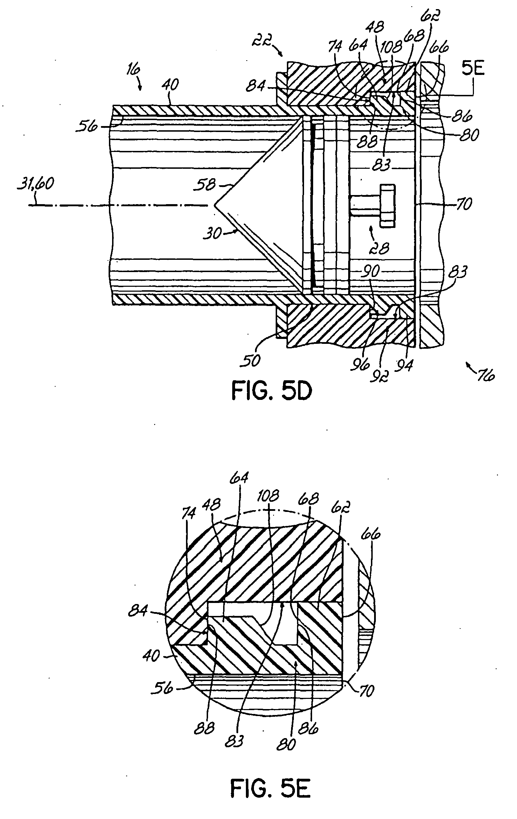

Referring now to Figs. 5A-5E, a third embodiment of a syringe 16 is shown having at least one syringe mating section (a first syringe mating section 48) coupled to a body 40 of the syringe 16. This first syringe mating section 48 is adapted to engage an injector 10 and includes a first flange 62 and a second flange 64, each extending outwardly from the body 40 along a plane substantially perpendicular to the longitudinal axis 60 of the body 40. In particular, the first flange 62 includes a rearward surface 66 and a forward surface 68. The rearward surface 66 of the first flange 62 may be flush or substantially flush with a proximal end 70 of the syringe 16. The second flange 64 is located distally of the first flange 62 and includes a rearward surface 72 and a forward surface 74, with the rearward surface 72 being spaced from the forward surface 68 of the first flange 62. Other embodiments of the syringe 16 may exhibit other appropriate positionings of the first and second flanges 62, 64 along the surface of the body 40. The first flange 62 has a cross-section in the form of a parallelogram, and the second flange 64 includes a tapered surface 108 disposed at a non-perpendicular and non-parallel angle relative to the longitudinal axis 60 of the syringe 16. Although the first flange 62 is shown as having a parallelogram cross-section and the second flange 64 is shown as including the tapered surface 108, it will be recognized by those skilled in the art that the first flange 62 may include a tapered surface 108, and the second flange 64 may have a cross-section of a parallelogram.

Like the embodiments described above, the syringe mating section 48, as illustrated in Figs. 5A-5E, encompasses an arcuate length generally equal to approximately one quarter of the circumference (that is, about 90°) of the body 40. However, it will be understood by those skilled in the art that the syringe mating section 48 could encompass an arcuate length of greater or lesser than about 90°.

As can be seen in Figs. 5B through 5E, the syringe 16 including the first syringe mating section 48 can be mounted to injectors of more than one design. A first injector 76, having a face plate 22 with a first injector mating section 52, is depicted in Figs. 5B-5C, and a second injector 78, having a face plate 22 with a second injector mating section 80, is depicted in Figs. 5D-5E. The injector mating section 52 of the first injector 76 (Figs. 5B-5C) is defined by a contour 82 that is complementary to the first syringe mating section 48. In other words, the contour 82 of the injector mating section 52 is closely aligned with and substantially matches a contour 84 defining the first syringe mating section 48. In particular, the injector mating section 52 includes a first groove 86 and a second groove 88 disposed in a surface 38 of the face plate 22. Thus, the first groove 86 has a cross-section in the shape of a parallelogram, and the second groove 88 has a tapered surface to complementarily match that of the second flange 64. When the syringe 16 is operatively connected to the face plate 22, the first flange 62 is received within the first groove 86 and the second flange 64 is received within the second groove 88. Again, those skilled in the art will recognize that cross-sectional shapes of the injector mating section grooves 86, 88 may be other than the shapes shown in the illustrated embodiment and may be dependent on the shape of the flanges 62, 64 of the syringe mating section 48.

The injector mating section 80 of the second injector 78 (Figs. 5D-5E) is defined by a contour 83 that cooperates with the first syringe mating section 48, but is not completely complementary to the first syringe mating section 48. In other words, the contour 83 does not substantially match the contour 84 of the first syringe mating section 48. However, the contour 83 is such that the injector mating section 80 does receive a first and second flanges 62, 64, in such manner that the first syringe mating section 48 and injector mating section 80 engage to operatively connect the syringe 16 to the second injector 78. In particular, the injector mating section 80 includes a single groove 90 having a dimension parallel to the longitudinal axis 60 of the syringe 16 that receives both the first flange 62 and the second flange 64 of the first syringe mating section 48. Further, while the single groove 90 of the illustrated embodiment is depicted as having a rectangular cross-section, those skilled in the art will recognize that a single groove of any alternate shape may suffice, provided it can receive the syringe mating section 48 to form a connection between the syringe 16 and second injector 78. Thus, like the embodiments described above, the syringe 16 of the embodiment of Figs. 5A-5E can be used with different injectors 76, 78 having different injector mating sections 52, 80, thereby increasing the versatility of the syringe 16.

Like the embodiment illustrated in Figs. 3A-3E above, in the embodiment of Figs. 5A-5E, each of the first and second flanges 62, 64 may extend only partially circumferentially around the rearward end 50 of body 40. Further, the syringe 16 of the illustrated embodiment includes a second syringe mating section 92 operatively coupled to the body 40, and positioned substantially opposite the first syringe mating section 48. Thus, as illustrated, the first and second syringe mating sections 48, 92 combined encompass an arcuate length generally equal to at least approximately one half of the circumference (i.e., about 180°) of the body 40 (although it will be recognized by those skilled in the art that the mating sections 48, 92 may encompass an arcuate length other than about one-half of the circumference of the body 40, and that there may be more than first and second mating sections). And while the illustrated embodiment does not depict any discontinuity or cavity in the syringe mating sections 48, 92, those skilled in the art will recognize that the syringe mating section s 48, 92 may exhibit such a discontinuity and/or cavity. And while the syringe mating section s 48, 92 are shown as engaging a face plate 22, it may be adapted to engage other portions of the injector 10, such as the housing 18 of the injector 10.

The syringe mating sections 48, 92 of the embodiment of Figs. 5A-5E may allow the syringe 16 to withstand greater shear forces than conventional syringe mating sections. This may reduce the failure rate of the syringes 16 as compared to conventional syringes. For example, a syringe mating section 48 of the embodiment illustrated in Figs. 5A-5E may have about .310 square inch of area for shear resistance.

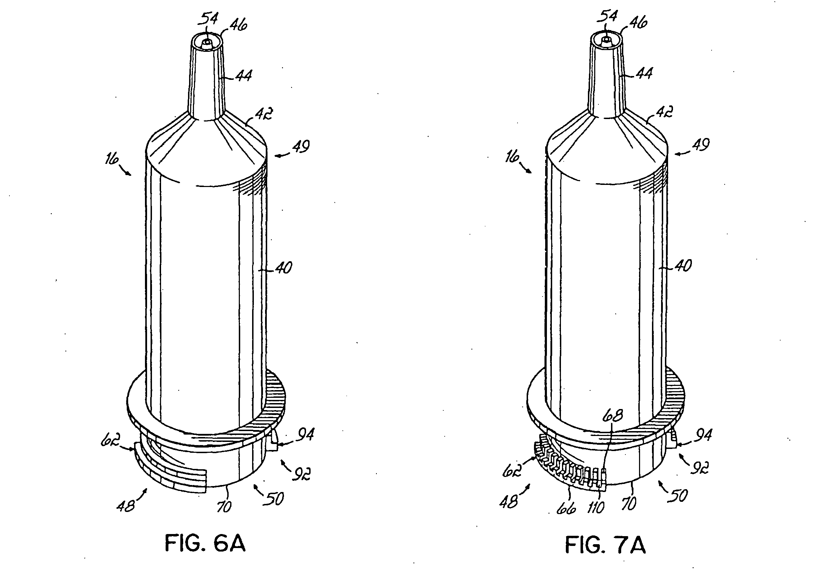

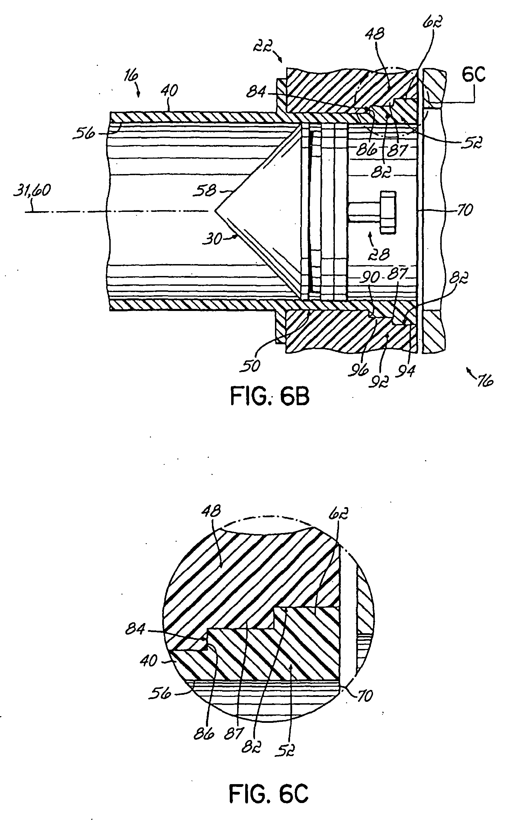

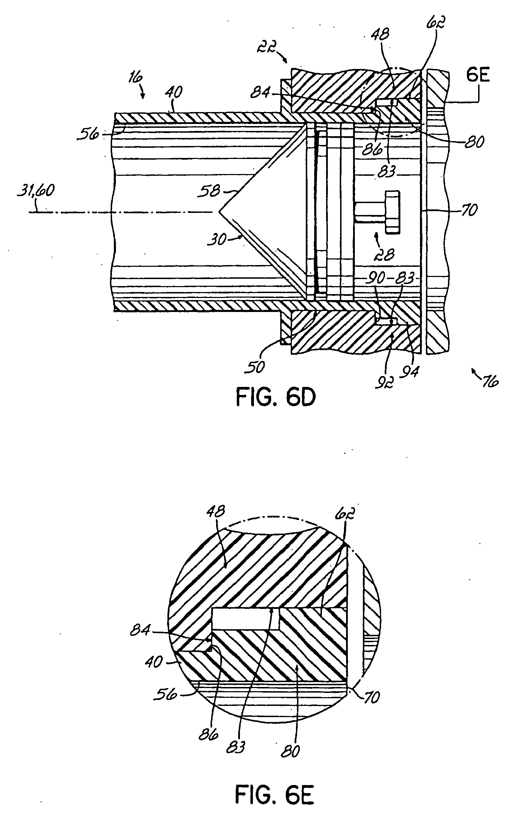

Referring now to Figs. 6A-6E, a fourth embodiment of a syringe 16 is shown having at least one syringe mating section (a first syringe mating section 48) coupled to the body 40 of the syringe 16. The first syringe mating section 48 is adapted to operatively engage an injector 10 and includes at least one flange 62 extending outwardly from the body 40 along a plane substantially perpendicular to the longitudinal axis 60 of the body 40. Further, this flange 62 exhibits an L-shaped cross-section and is positioned toward the proximal end 70 of the body 40 of the syringe 16. Other embodiments of the syringe 16 may exhibit other appropriate positionings of the flange 62 along the surface of the body 40.

The first syringe mating section 48 shown in Figs. 6A-6E encompasses an arcuate length generally equal to approximately one quarter of the circumference (that is, about 90°) of the body 40. However, as above, it will be understood by those skilled in the art that the syringe mating section 48 could encompass an arcuate length of greater or lesser than about 90°.

As can be seen in Figs. 6B through 6E, the syringe 16 including the first syringe mating section 48 can be mounted to injectors exhibiting different designs. A first injector 76, having a face plate 22 with a first injector mating section 52, is depicted in Figs. 6B-6C, and a second injector 78, having a face plate 22 with a second injector mating section 80, is depicted in Figs. 6D-6E. The injector mating section 52 of the first injector 76 (Figs. 6B-6C) is defined by a contour 82 that is complementary to the first syringe mating section 48. In other words, the contour 82 of the injector mating section 52 is closely aligned with and substantially matches a contour 84 defining the first syringe mating section 48. In particular, the injector mating section 52 includes a first groove 86 disposed in a surface 38 of the face plate 22. This groove 86 includes an L-shaped contour 82 that complimentarily cooperates with the L-shaped flange 62. When the syringe 16 is operatively connected to the face plate 22, the flange 62 having an L-shaped cross-section is received within the first groove 86 such that the syringe 16 is operatively connected to the injector 10.

The injector mating section 80 of the second injector 78 (Figs. 6D-6E) is defined by a contour 83 that cooperates with the first syringe mating section 48, but is not completely complementary to the first syringe mating section 48. In other words, the contour 83 does not substantially match the contour 84 of the first syringe mating section 48. However, the contour 83 is such that the injector mating section 80 does receive an L-shaped flange 62 in such manner that the first syringe mating section 48 and injector mating section 80 engage to operatively connect the syringe 16 to the second injector 78. In particular, the injector mating section 80 includes a single groove 90 having a dimension parallel to the longitudinal axis 60 of the syringe 16 that receives the flange 62 of the first syringe mating section 48. Further, while the single groove 90 of the illustrated embodiment is depicted as having a rectangular cross-section, those skilled in the art will recognize that a single groove of any alternate shape may suffice, provided it can receive the syringe mating section 48 to form a connection between the syringe 16 and second injector 78. Thus, the syringe 16 of the embodiment of Figs. 6A-6E can be used with different injectors 76, 78 having different injector mating sections 52, 80, thereby increasing the versatility of the syringe 16.

In the syringe embodiment of Figs. 6A-6E, the flange 62 may extend only partially circumferentially around the body 40. The syringe 16 of the illustrated embodiment further includes a second syringe mating section 92 operatively coupled to the body 40 and positioned substantially opposite the first syringe mating section 48. Thus, the first and second syringe mating sections 48, 92 combined encompass an arcuate length generally equal to approximately one half of the circumference (i.e., about 180°) of the body 40 (although it will be recognized by those skilled in the art that the mating sections 48, 92 may encompass an arcuate length other than about one-half of the circumference of the body 40, and that there may be more than first and second mating sections). And while the syringe mating section 92 is shown as engaging a face plate 22, it may be adapted to engage other portions of an injector, such as a housing of an injector.

The syringe mating sections 48, 92 of the embodiment of Figs. 6A-6E may allow the syringe 16 to withstand greater shear forces than conventional syringe mating sections. This may reduce the failure rate of the syringes 16 of the present invention as compared to conventional syringes. For example, a syringe mating section 48 of the embodiment illustrated in Figs. 6A-6E may have about 0.554 square inch of area for shear resistance.

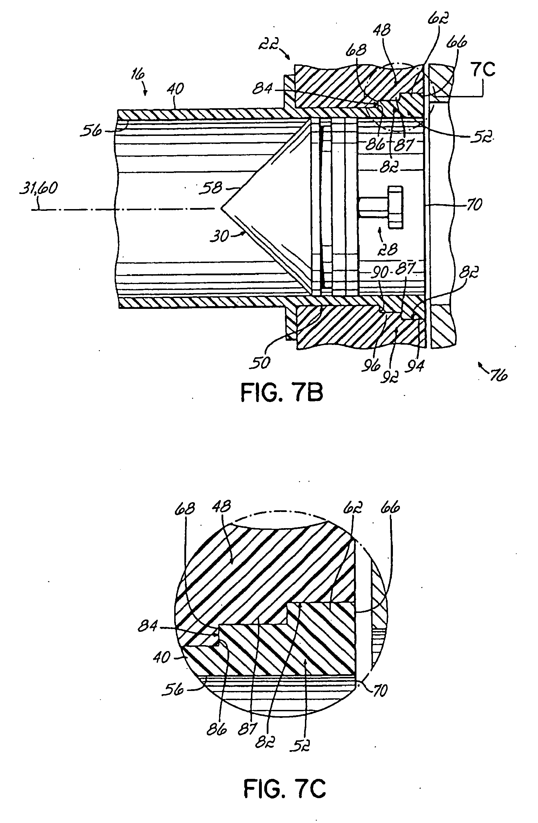

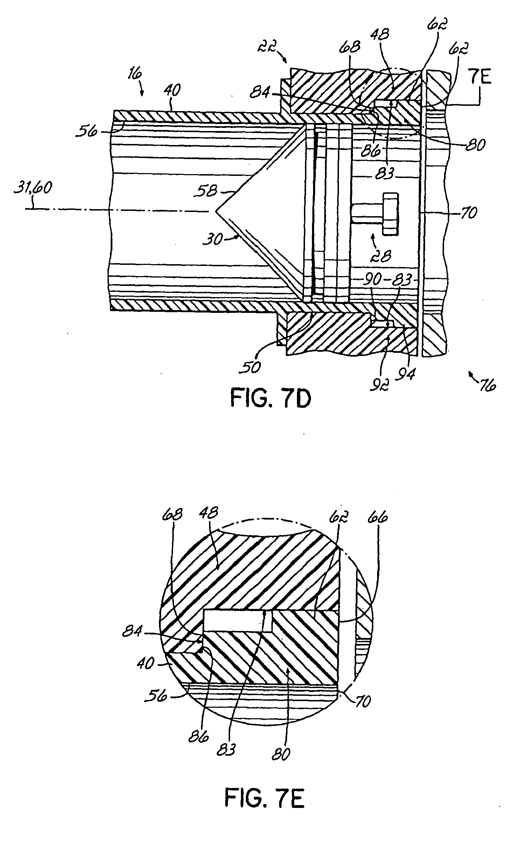

Referring now to Figs. 7A-7E, a fifth embodiment of a syringe 16 is shown having at least one syringe mating section (a first syringe mating section 48) coupled to a body 40 of the syringe 16. This first syringe mating section 48 is adapted to operatively engage an injector 10 and includes at least one flange 62 extending outwardly from the body 40 along a plane substantially perpendicular to the longitudinal axis 60 of the body 40. The flange 62 exhibits an L-shaped cross-section and is located toward the rearward end of the body 40. Other embodiments may exhibit other appropriate positionings of the L-shaped flange along the surface of the body 40. The flange 62 includes a discontinuity 110 along its length. In particular, the flange 62 includes a plurality of discontinuities 110 along its length. The discontinuities 110 are shown as notches in the flange 62. However, those skilled in the art will recognize that a discontinuity can refer to features other than a notch (e.g., a gap or an undulating contour). The discontinuities 110 of the illustrated embodiment are shown as extending from a forward surface 68 toward a rearward surface 66 of the flange 62. Those skilled in the art will recognize that this is not crucial to the invention, and that the discontinuities may extend from the rearward surface 66 toward the forward surface 68.

The syringe mating section 48 of Figs. 7A-7E encompasses an arcuate length generally equal to approximately one quarter of the circumference (that is, approximately 90°) of the body 40. However, it will be understood by those skilled in the art that the syringe mating section 48 could encompass an arcuate length of greater or less than about 90°.

As can be seen in Figs. 7B through 7E, the syringe 16 including the first syringe mating section 48 can be mounted to injectors of more than one design. A first injector 76, having a face plate 22 with a first injector mating section 52, is depicted in Figs. 7B-7C, and a second injector 78, having a face plate 22 with a second injector mating section 80, is depicted in Figs. 7D-7E. The injector mating section 52 of the first injector 76 (Figs. 7B-7C) is defined by a contour 82 that is substantially complementary to the first syringe mating section 48. As above, the contour 82 of the injector mating section 52 is closely aligned with and substantially matches a contour 84 defining the first syringe mating section 48. In particular, the injector mating section 52, as illustrated, includes a first groove 86 disposed in a surface 38 of the face plate 22. This groove 86, as illustrated, includes an L-shaped contour 82 that cooperates with the L-shaped flange 62. When the syringe 16 is operatively connected to the face plate 22, the flange 62 having an L-shaped cross-section is received within the first groove 86.

The injector mating section 80 of the second injector 78 (Figs. 7D-7E) is defined by a contour 83 that cooperates with the first syringe mating section 48, but is not completely complementary to the first syringe mating section 48. In other words, the contour 83 does not substantially match the contour 84 of the first syringe mating section 48. However, the contour 83 is such that the injector mating section 80 does receive an L-shaped flange 62 in such manner that the first syringe mating section 48 and injector mating section 80 engage to operatively connect the syringe 16 to the second injector 78. In particular, the injector mating section 80 includes a single groove 90 having a dimension parallel to the longitudinal axis 60 of the syringe 16 that receives the flange 62 of the first syringe mating section 48. Further, while the single groove 90 of the illustrated embodiment is depicted as having a rectangular cross-section, those skilled in the art will recognize that a single groove of any alternate shape may suffice, provided it can receive the syringe mating section to form a connection between the syringe 16 and second injector 78. Thus, the syringe 16 of the embodiment of Figs. 7A-7E can be used with different injectors 76, 78 having different injector mating sections 52, 80, thereby increasing the versatility of the syringe 16.

Further, like the embodiment illustrated in Figs. 3A-3E above, in the syringe embodiment of Figs. 7A-7E, the flange 62 may extend only partially circumferentially around the rearward end 50 of body 40. Further, the syringe 16 of the illustrated embodiment further includes a second syringe mating section 92 operatively coupled to the body 40, and positioned substantially opposite the first syringe mating section 48. Thus, as illustrated, the first and second syringe mating sections 48, 92 combined encompass an arcuate length generally equal to at least approximately one half of the circumference (i.e., about 180°) of the body 40 (although it will be recognized by those skilled in the art that the mating sections 48, 92 may encompass an arcuate length other than about one-half of the circumference of the body 40, and that there may be more than first and second mating sections). And while the syringe mating section 92 is shown as engaging a face plate 22, it may be adapted to engage other portions of the injector 10, such as the housing 18 of the injector 10.

Further, the syringe mating sections 48, 92 of the embodiment of Figs. 7A-7E allows the syringe 16 to withstand greater shear forces than previous syringe mating sections. This reduces the failure rate of the syringes 16 of the present invention as compared to previous syringes. For example, the syringe mating section 48 of the embodiment illustrated in Figs. 7A-7E may have about .291 square inch of area for shear resistance, for example.

Additional advantages and modifications will readily appear to those skilled in the art. The invention in its broader aspects is therefore not limited to the specific details, representative apparatus and methods, and illustrative examples shown and described. Accordingly, departures may be made from such details without departing from the scope or spirit of Applicants general inventive concept.

## Claims

1. A syringe comprising:
a body (40) having a longitudinal axis and including a forward end and a rearward end, the forward end having a fluid discharge section (44) defined therein; and
a first syringe mating section (48) coupled to the body (40), the first syringe mating section (48) comprising first and second flanges (62, 64) each extending outwardly from the body away from the longitudinal axis along a plane substantially perpendicular to the longitudinal axis, wherein one of said flanges (62, 64) has a cross-section in the form of a parallelogram, and wherein the other of said flanges (62, 64) including a tapered surface (108) relative to the longitudinal axis.

2. A syringe according to claim 1, wherein said one of said flanges is proximal of said other of said flanges.

3. A syringe according to claim 1 or claim 2, wherein the most proximal flange is substantially flush with the proximal end of said body (40).

4. A syringe according to any of claims 1-3, wherein said first flange (62) has an arcuate length of approximately one quarter of the circumference of said body (40).

5. A syringe according to any of claims 1-4, wherein said second flange (64) has an arcuate length of approximately one quarter of the circumference of said body (40).

6. The syringe of any one of claims 1-5, wherein at least one of said flanges (62, 64) exhibits at least one discontinuity along its length.

7. The syringe of claim 6, wherein said discontinuity comprises a cavity disposed in said flange.

8. The syringe of any preceding claim, wherein the first syringe mating section (48) has an area of at least about 1.3mm².

9. An injection system comprising:
a medical fluid injector; and
a syringe of any preceding claim mounted to the injector, wherein the first syringe mating section (48) is engaged with a face plate of the injector.

10. The injection system of claim 9, wherein the faceplate (22) has an injector mating section (52) defining a contour (82) that confronts the first syringe mating section (48).

11. The injection system of claim 10, wherein the contour (82) defines at least one groove (86, 88).

12. The injection system of claim 11, wherein said contour defines two grooves (86, 88), one each for engagement with a respective one of said flanges (62, 64).

13. The injection system of claim 12, wherein said grooves (86, 88) are complimentary in contour to the respective flanges (62, 64).

14. A method of releasably interconnecting a syringe of any one of claims 1-13 with a medical fluid injector, the method comprising:
aligning the first syringe mating section (48) with an injector mating section (52) of the medical fluid injector; and
rotating the syringe relative to the medical fluid injector to place the first syringe mating section and the injector mating section in an engaged relationship.

15. A method according to claim 14, wherein relative rotation of said syringe tightly engages the tapered surface (108) of said other flange (64) with a tapered groove (88) of said injector mating section (52).
